# EUROPEAN PATENT APPLICATION

(11) **EP 1 875 872 A1**
(43) Date of publication of application: **09.01.2008**
(21) Application number: 06731030.0
(22) Date of filing: 04.04.2006
(51) Int. Cl.: A61B 17/28

(54) **TREATMENT INSTRUMENT FOR ENDOSCOPE**

(30) Priority: 18.04.2005 JP 2005120086
(71) Applicant: OLYMPUS MEDICAL SYSTEMS CORP., Shibuya-ku Tokyo 151-0072 (JP)
(72) Inventor: KIMURA, Hiroshi, Saitama-ken (JP); IWANAGA, Naoki, Urawa-ku,Saitama-shi (JP)
(74) Representative: von Hellfeld, Axel
(86) International application number: PCT/JP2006/307082
(87) International publication number: WO 2006/114989

(57) **Abstract**

Endoscopic biopsy forceps as one of endoscopic treatment tools of the present invention include a flexible insertion section. The proximal end portion of the insertion section is received in an operating section. A closely wound coil is exposed to the proximal end portion of the insertion section, and the proximal end portion of the coil is enlarged in diameter to form an enlarged-diameter portion. A protrusion is formed in an operation section body, and the enlarged-diameter portion of the coil is locked by the protrusion to restrict the movement of the coil, thereby preventing slipping off of the insertion section.

## Description

### TECHNICAL FIELD

The present invention relates to an endoscopic treatment tool that performs a treatment on living tissues while being inserted into a channel of an endoscope.
This application is based upon and claims the benefit of priority from the prior Japanese Patent Application No. 2005-120086, filed April 18, 2005, the entire contents of which are incorporated herein by reference.

### BACKGROUND ART

An endoscopic treatment tool is provided with a flexible insertion section that can be inserted into a channel of an endoscope, and the insertion section is inserted into a sheath so that an operating wire is freely moved forward and backward in the axial direction of the insertion section. An operating wire is connected to an operating section that is disposed in a proximally disposed end (a proximal end disposed close to an operator's hand) of the insertion section and is also connected to a treatment section that is disposed in a distally disposed end (a distal end) of the insertion section. For this reason, by operating the operating section to move the operating wire forward and backward, it is possible to drive the treatment section.

As the means for attaching the insertion section to the operating section, there is known a method of inserting and fixing the proximal end portion of the insertion section into a groove prepared in an operating section body of the operating section (see Patent Document 1, for example). In the endoscopic treatment tool disclosed in Patent Document 1, a cylindrical stopper is welded and fixed to the proximal end of the sheath of the operating section, and a step is formed in the groove of the operating section body so as to be engaged with the stopper. Therefore, when the insertion section is inserted into the groove in a direction from the open end of the groove while closing the open end with a body cover, the stopper is engaged with the step, thus preventing slipping off of the insertion section.
[Patent Document 1] Japanese Unexamined Patent Application, First Publication No. 2000-175928

### DISCLOSURE OF THE INVENTION

### PROBLEM TO BE SOLVED BY THE INVENTION

However, such an endoscopic treatment tool has a problem in that the number of components may increase thus increasing cost. Moreover, it is necessary to fix the stopper to a coil strongly enough to sustain the force of pulling the insertion section, thus increasing the number of processes and resulting in poor production efficiency.
The present invention is contrived to solve the above-mentioned problems. An object of the present invention is to enable attaching of an insertion section to an operating section with a simple construction.

### MEANS FOR SOLVING THE PROBLEM

In order to accomplish the above-mentioned object, according to a first aspect of the invention, there is provided an endoscopic treatment tool including: an insertion section that has flexibility to enable its insertion into a channel of an endoscope; a treatment section that is disposed on a distal end side of the insertion section, and which performs a treatment on a living body; and an operating section that is disposed in a proximal end portion of the insertion section, and which performs an operation of the treatment section, wherein an operating wire is inserted into the insertion section so as to be freely moved forward and backward, and the operating wire connects the operating section to a driving portion of the treatment section, the operating section including: a receiving section that is configured to receive the proximal end portion of the insertion section therein; and a protrusion that is formed so as to decrease the width of the receiving section, the insertion section having a wire coil, and wherein an enlarged-diameter portion is formed in the proximal end portion of the coil so as to be engaged with the protrusion.
In the endoscopic treatment tool, when the coil is inserted into the receiving section formed in the operating section, the enlarged-diameter portion of the coil is inserted into the protrusion in an engaging manner. In cases such as treatment, when a relative pulling force is applied from the operating section to the insertion section, the enlarged-diameter portion of the coil is engaged with the protrusion so as to restrict the movement of the insertion section.

According to a second aspect of the invention, there is provided an endoscopic treatment tool including: an insertion section that has flexibility to enable its insertion into a channel of an endoscope; a treatment section that is disposed on a distal end side of the insertion section, and which performs a treatment on a living body; and an operating section that is disposed in a proximal end portion of the insertion section, and which performs an operation of the treatment section, wherein an operating wire is inserted into the insertion section so as to be freely moved forward and backward, and the operating wire connects the operating section to a driving portion of the treatment section, the insertion section including a wire coil, and wherein a pressing member is inserted into the proximal end of the coil so as to enlarge a winding diameter of the coil, thereby forming an enlarged-diameter portion in the coil so as to be engaged with the operating section.
In the endoscopic treatment tool, the pressing member is extended into the coil, thus enlarging the winding diameter of the coil. At this time, since the diameter of the coil wire does not change much, the strength of the coil is not decreased. Moreover, since the enlarged-diameter portion is engaged with the operating section, it is possible to prevent slipping off of the insertion section.

### EFFECTS OF THE INVENTION

According to the present invention, since the enlarged-diameter portion is formed in the coil so as to be engaged with the operating section, it is unnecessary to fixedly attach an additional member to the coil. Therefore, it is possible to decrease the number of components and thus to decrease the number of processes required for fixation of the additional member. Accordingly, it is possible to decrease the production cost and to improve the production efficiency.

### BRIEF DESCRIPTION OF THE DRAWINGS

FIG. 1 is a diagram showing the configuration of an endoscopic treatment tool according to an embodiment of the present invention.
FIG. 2 is a sectional view taken along the line A-A of FIG. 1.
FIG. 3 is a sectional view taken along the line B-B of FIG. 1, showing the shape of an operating section body.
FIG. 4 is a sectional view taken along the line B-B of FIG. 1, showing the state in which an operating section is attached.
FIG. 5 is a diagram for explaining a method for forming an enlarged-diameter portion in a coil.
FIG. 6 is a diagram showing the state in which the enlarged-diameter portion is formed in the coil.

### DESCRIPTION OF THE REFERENCE SYMBOLS

1: ENDOSCOPIC BIOPSY FORCEPS (ENDOSCOPIC TREATMENT TOOL)
2: INSERTION SECTION
3: OPERATING SECTION
4: TREATMENT SECTION
10: COIL
10A: ENLARGED-DIAMETER PORTION
13, 14: OPERATING WIRE
17, 18: BIOPSY CUP (DRIVING SECTION)
27: PROTRUSION
30: RECEIVING SECTION
54: PUNCH (PRESSING MEMBER)

### BEST MODE FOR CARRYING OUT THE INVENTION

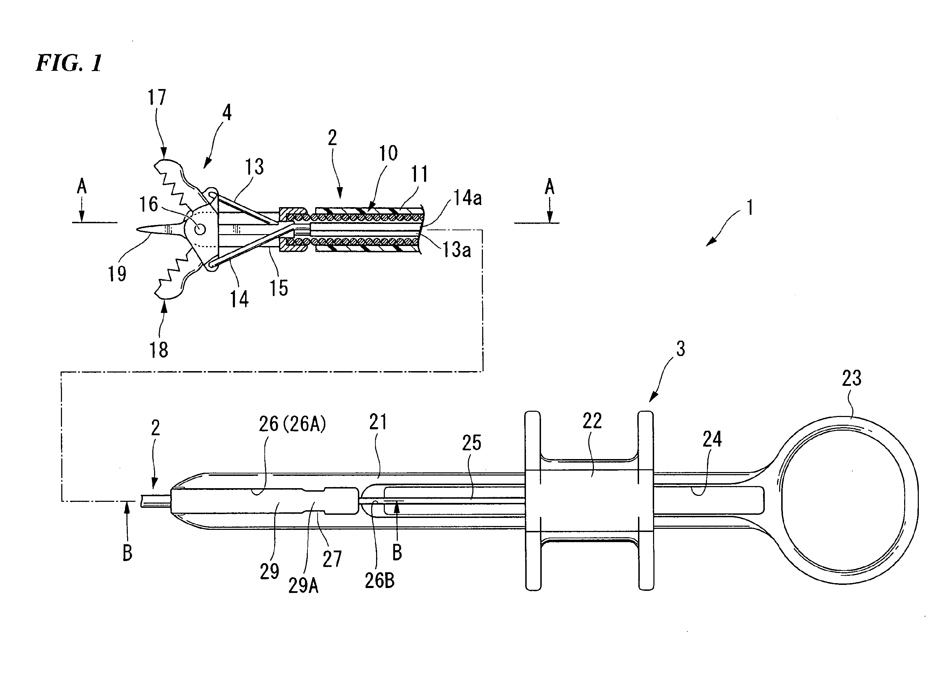

Hereinafter, an embodiment of the present invention will be described with reference to the accompanying drawings. The endoscopic treatment tool of the embodiment will be described and illustrated as being endoscopic biopsy forceps, but the invention is not limited to this.
As shown in FIG. 1, an endoscopic biopsy forceps 1 is provided with an elongated insertion section 2; an operating section 3 that is disposed in a proximally disposed end (a proximal end) of the insertion section 2, and which is used outside the body; and a treatment section 4 that is disposed in a distally disposed end (a distal end) of the insertion section 2, and which performs a treatment inside the body.

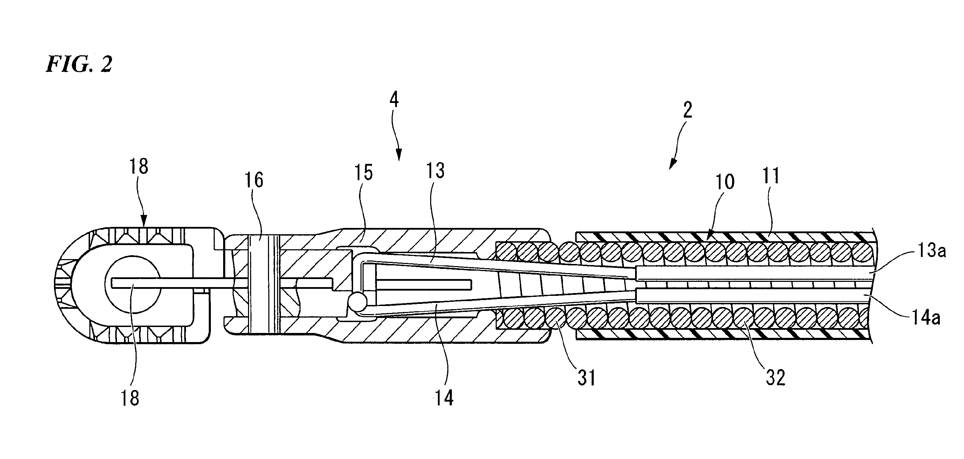

As shown in FIG. 2, the insertion section 2 is provided with a sheath in which an outside tube 11 is produced by applying a thermal shrinkage tube or performing tubing molding on the outer surface of a closely wound coil 10. Two operating wires 13 and 14 are inserted into the coil 10 so as to be freely moved forward and backward. The two operating wires 13 and 14 are coated with individual coating materials 13A and 14A, respectively. The outside tube 11 is not an essential component and may not be used.

As a material for the outside tube 11, there can be used, for example, high density polyethylene, a mixture of high density polyethylene and low density polyethylene, a mixture of low density polyethylene and polypropylene, and the like, which are available at a low cost and excellent in insertion ability through the channel of the endoscope. The outer surface of the outside tube 11 may have such a shape as is produced by transfer of the surface irregularity of the coil 10 to the outer surface. Alternatively, a plurality of parallel ridges may be formed on the outer surface of the outside tube 11 so as to extend in the axial direction of the outside tube 11.

A cup holding member 15 is provided to the proximal end portion of the coil 10 so as to be fitted to the coil 10. The cup holding member 15 is fixed to the coil 10 by means of laser welding, welding, soldering, caulking or the like. A pin 16 is inserted into the vicinity of the distal end portion of the cup holding member 15. A pair of biopsy cups 17 and 18 (driving portion) are disposed so as to be freely rotatable about the pin 16. Respective proximal end portions of the biopsy cups 17 and 18 are connected to the corresponding one of the operating wires 13 and 14. With the forward and backward movement of the operating wires 13 and 14, the pair of biopsy cups 17 and 18 is opened and closed. A needle 19 is disposed between the biopsy cups 17 and 18. The needle 19 is fixed to the cup holding member 15, and a sharp distal end portion of the needle 19 is protruded out from the cup holding member 15.

The operating section 3 is mounted on an operating section body 21 so that a slider 22 is freely moved forward and backward. The insertion section 2 is inserted into the distal end of the operating section body 21, and a finger hook ring 23 is provided to the proximal end of the operating section body 21. A slit 24 is formed in the operating section body 21 so as to extend from the vicinity of the ring 23 to the distal end portion of the operating section body 21. The slider 22 is mounted in the slit 24. The proximal end portions of the operating wires 13 and 14 are fixed to the slider 22. As the slider 22 is moved along the slit 24, the operating wires 13 and 14 are moved forward and backward. A hard operating pipe 25 is inserted between the distal end portion of the slit 24 and the slider 22. By allowing the operating wires 13 and 14 to be inserted into the operating pipe 25, it is possible to prevent bending (buckling) of the operating wires 13 and 14 at the time of the movement of the slider 22.

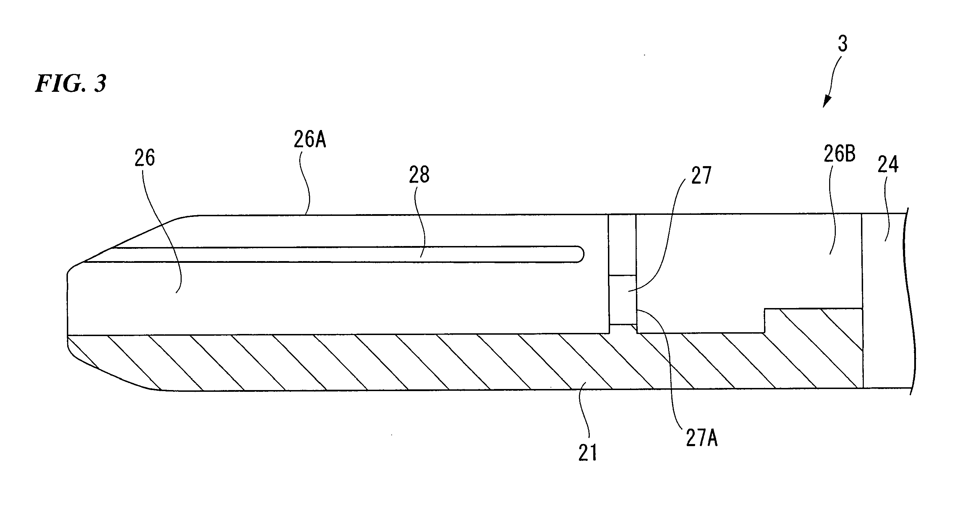

As shown in FIG. 3, a receiving groove 26 is formed in the operating section body 21 so as to extend from the distal end surface of the operating section body 21 to the slit 24. The receiving groove 26 has an open end 26A that is disposed on one side portion of the operating section body 21. The receiving groove 26 has such a width that the insertion section can be inserted therein. On a portion of the receiving groove 26 disposed closer to the slit 24 in the longitudinal direction than the middle portion, a protrusion 27 is formed so as to decrease the width and depth of the receiving groove 26. The proximal end portion of the receiving groove 26 is formed as a communicating groove 26B that communicates with the slit 24. The communicating groove 26B has such a width that the operating wires 13 and 14 and the operating pipe 25 are freely moved forward and backward into the groove but the coil is not able to extend into the groove. A concave portion 28 is formed in a wall portion of the receiving groove 26 so as to extend from the distal end in a direction parallel to the longitudinal direction of the receiving groove 26. As shown in FIG. 1, when the insertion section 2 is attached to the operating section 3, the insertion section 2 is first inserted into the receiving groove 26 and thereafter a body cover 29 is mounted in the receiving groove 26, thereby closing the open end 26A of the receiving groove 26. A concave portion 29A is formed in the body cover 29. By allowing the concave portion to be engaged with the protrusion 27, it is possible to restrict the movement in the longitudinal direction of the body cover 29. In addition, by allowing a concave portion 28 shown in FIG. 3 to be engaged with a claw (not shown), it is possible to restrict the movement in a direction perpendicular to the longitudinal direction of the body cover 29.

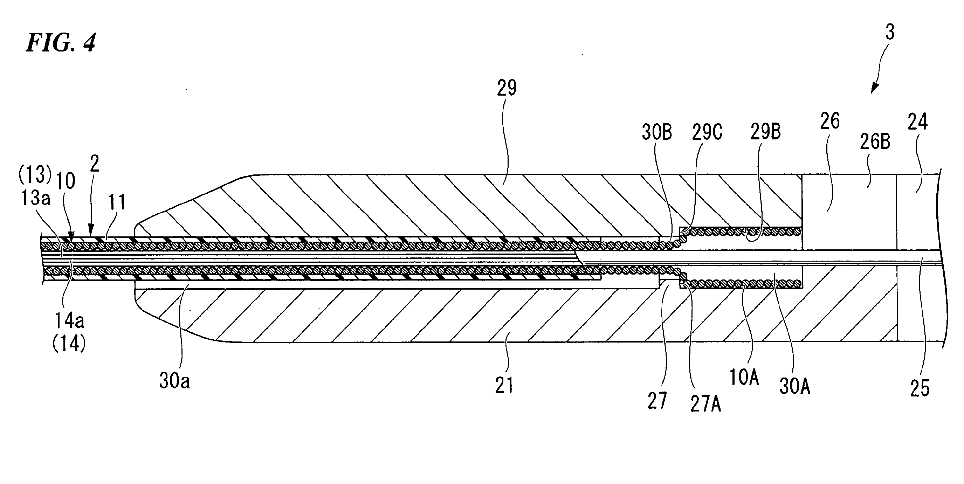

As shown in FIG. 4, when the body cover 29 is mounted in the receiving groove 26, an elongated receiving section 30 is formed such that the insertion section 2 is received in the operating section 3. A cutout 29B is formed in the proximal end portion of the body cover 29. Thus, in the receiving section 30, a proximal end portion 30A disposed closer to the slit than the protrusion 27 is formed with a larger width than other portions of the receiving section 30. In addition, in the insertion section 2, the coil 10 is protruded closer to the proximal end than the outside tube 11, and an enlarged-diameter portion 10A having an enlarged winding diameter is formed in the proximal end portion of the coil 10. The enlarged-diameter portion 10A has such a size that the portion 10A can go into the proximal end portion 30A of the receiving section but cannot pass through a narrow-width portion 30B that is narrowed by the protrusion 27.

The coil 10, the operating wires 13 and 14 and the needle 19 are made of a metallic material. For example, they can be made of the following metals or alloys thereof: stainless, aluminum, nickel, brass, titanium, iron, phosphor bronze, tungsten, gold, silver, copper, and the like.

The operating section body 21 and the slider 22 are formed of a resin material that can be treated by thermal sterilization. For example, they can be formed of polysulfone, polyphenylsulfone, polyetherimide, polytetrafluoroethylene (PTFE), tetrafluoroethylene-perfluoroalkoxy ethylene resin (PFA), tetrafluoroethylene-hexafluoropropylene resin (FEP), polyacetal, poly(ether-ether-ketone), and the like.

Further, since the operating section body 21 and the slider 22 can be made to be discarded after usage, they can be fabricated with low cost resin materials. For example, they can be fabricated with resin materials such as polyolefin, polycarbonate, acrylonitrile butadiene-styrene resin, polyamide, vinyl chloride, latex, polypropylene, polyethylene-terephthalate, ethylene-vinyl acetate copolymer, or natural rubber, mixtures of the resin materials, and cross-linked resins produced by irradiating the above exemplified resins with an electron beam.

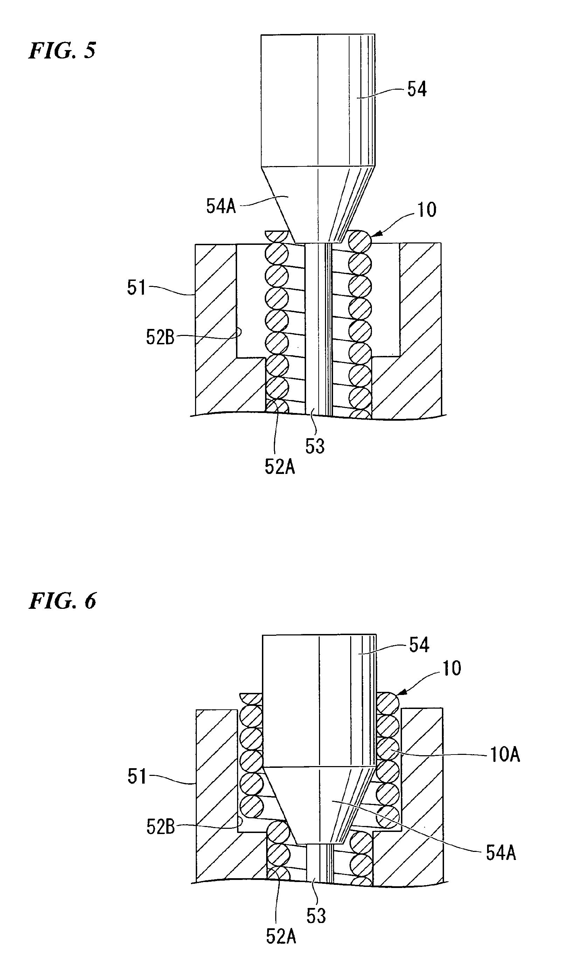

Next, a method for forming the enlarged-diameter portion 10A in the coil 10 of the insertion section 2 will be described.
First, as shown in FIG. 5, the coil 10 is inserted into a hole of a die 51. The diameter in a lower portion 52A of the hole 52 is made substantially equal to the outer diameter of the coil 10 while the diameter in an upper portion 52B of the hole 52 is made larger than the outer diameter of the coil 10. A step is formed between the lower portion 52A and the upper portion 52B of the hole 52 and the diameter of the hole 52 changes in a discrete manner. Here, the diameter in the lower portion 52A is smaller than the width of the narrow-width portion 30B formed in the operating section body 21. The diameter in the upper portion 52B is substantially equal to the width of the proximal end portion 30A of the receiving section formed in the operating section body 21. In addition, the length of the upper portion 52B is smaller than the distance between the protrusion 27 and the communicating groove 26B.

When the coil 10 is inserted into the hole 52, a core metal 53 that is sufficiently thinner than the inner diameter of the coil is inserted into the coil 10 from the upper side, and a punch 54 as the pressing member is gradually inserted into the coil 10 while being guided by the core metal 53. A distal end portion 54A of the punch 54 has a substantially conical trapezoid shape in which a lower end having a diameter smaller than the inner diameter of the coil 10, and in which the diameter of the distal end portion 54A is gradually enlarged as it goes upward from the lower end. The maximum outer diameter of the punch 54 is larger than the diameter in the lower portion 52A of the hole 52 but is smaller than the diameter in the upper portion 52B. More specifically, it is preferable that The maximum outer diameter of the punch 54 is larger than the width of the narrow-width portion 30B of the receiving section 30 formed in the operating section body 21 and has such a size that the punch 54 can press the coil 20 against the inner periphery in the upper portion 52B of the hole 52.
When the punch 54 is inserted into the coil 10, the end portion of the coil 10 is pressed against the distal end portion 54A of the punch 54 in a direction from the inner side toward the outer side, thereby gradually enlarging the winding diameter of the coil without varying the wire's diameter of the coil 10. As a result, as shown in FIG. 6, the outer diameter of the coil 10 is increased, thus forming the enlarged-diameter portion 10A in the end portion of the coil 10. At this time, when the outer diameter of the coil 10 is for example about 2 mm, the diameter in the enlarged-diameter portion 10A becomes about 4 mm. In the case of forming the enlarged-diameter portion 10A, the punch 54 may be directly inserted into the coil without the use of the core metal 53.

When the operating section 3 of the endoscopic biopsy forceps 1 is assembled, the operating pipe 25 is inserted into the insertion section 2 in a direction from the proximal end, and thereafter the operating wires 13 and 14 are made to pass through the operating pipe 25 and then fixed to the slider 22. Then, the insertion section 2 is inserted into the receiving groove 26 of the operating section body 21. At this time, the operating pipe 25 is received in the communicating groove 26B while the enlarged-diameter portion 10A is received in a portion that is closer to the proximal end than the protrusion 27. Thereafter, the open end 26A of the receiving groove 26 is closed by the body cover 29.

When operating the endoscopic biopsy forceps 1, the insertion section 2 is inserted into the channel of the endoscope, and the treatment section 4 is made to be protruded from the distal end portion of the endoscope, thereby operating the operating section 3 in a state that the treatment section 4 is opposed to a target treatment portion. Specifically, the slider 22 is made to move forward so as to move forward the operating wires 13 and 14 that are fixed to the slider 22.
In this case, as shown in FIG. 4, when the coil 10 is pulled in a direction parallel to the longitudinal direction, the enlarged-diameter portion 10A is locked at both a proximal end surface 27A of the protrusion that is formed perpendicular to the longitudinal direction and the cutout surface 29B of the body cover 29 that is formed at the same height with the proximal end surface 27A. Accordingly, the movement of the coil 10 is restricted.

Therefore, since the operating wires 13 and 14 are freely movable forward but the movement of the coil 10 is restricted, the operating wires 13 and 14 are moved forward relative to the coil 10. As a result, the operating wires 13 and 14 are moved forward in a state that the cup holding member 15 fixed to the distal end of the coil 10 is in the stationary state. Accordingly, the pair of biopsy cups 17 and 18 that are connected to a corresponding one of the distal end portions of the operating wires 13 and 14 are rotated and opened. In this state, the biopsy cups 17 and 18 are pressed against the target treatment portion and the slider 22 is made to be moved backward. Since the operating wires 13 and 14 are made to be moved backward but the movement of the coil 10 is restricted, the pair of biopsy cups 17 and 18 are closed thus taking hold of the target treatment portion. In this state, as the insertion section 2 is pulled outside the body, the target treatment portion is extracted. Even in this case, the enlarged-diameter portion 10A of the coil 10 collides with the protrusion 27, thus preventing slipping off of the insertion section 2.
Therefore, the operating wires 13 and 14 are made to be moved forward relative to the coil 10. As a result, the pair of biopsy cups 17 and 18 that are connected to the corresponding one of the distal end portions of the operating wires 13 and 14 are opened. In this state, the biopsy cups 17 and 18 are pressed against the target treatment portion and the slider 22 is made to be moved backward. Since the operating wires 13 and 14 are made to be moved backward but the movement of the coil 10 is restricted, the pair of biopsy cups 17 and 18 are closed thus taking hold of the target treatment portion. In this state, the insertion section 2 is drawn outside the body, and the target treatment portion is extracted. Even in this case, the enlarged-diameter portion 10A of the coil 10 collides with the protrusion 27, thus preventing slipping off of the insertion section 2.

According to the above embodiment, since the enlarged-diameter portion 10A is formed by enlarging the diameter of the proximal end portion of the coil 10 of the insertion section 2, it is unnecessary to attach an additional member to the coil 10 in order to allow the insertion section to be engaged with the operating section body, which was conventionally required. Accordingly, it is possible to decrease the number of components. Since the enlarged-diameter portion 10A can be formed simply by pushing the punch 54 into the coil 10 that is inserted into the die 51, it is possible to simplify the fabrication. In addition, since the endoscopic biopsy forceps 1 can be assembled simply by making the coil be received in the receiving groove 26, it is possible to simplify the assembly. Accordingly, it is possible to simplify the production process and to decrease the production cost. In addition, since the enlarged-diameter portion 10A is formed in a portion of the coil 10, it is easy to secure a sufficient tensile strength.

The present invention is not limited to the above embodiment but may be applied to various other applications.
The formation method of the enlarged-diameter portion 10A is not limited to the pressing method based on the punch 54. For example, different-diameter portions may be formed at the time of winding the coil 10.
Furthermore, as long as the enlarged-diameter portion 10A is formed in a portion of the coil 10 where the coil 10 is received in the operating section 2, the enlarged-diameter portion 10A is not necessary to be formed in the proximal end portion of the coil 10.

## Claims

1. An endoscopic treatment tool comprising:
an insertion section that has flexibility to enable its insertion into a channel of an endoscope;
a treatment section that is disposed on a distal end side of the insertion section, and which performs a treatment on a living body; and
an operating section that is disposed in a proximal end portion of the insertion section, and which performs an operation of the treatment section,
wherein an operating wire is inserted into the insertion section so as to be freely moved forward and backward, and
the operating wire connects the operating section to a driving portion of the treatment section, the operating section comprising:
a receiving section that is configured to receive the proximal end portion of the insertion section therein; and
a protrusion that is formed so as to decrease the width of the receiving section, the insertion section comprising a wire coil, and
wherein an enlarged-diameter portion is formed in the proximal end portion of the coil so as to be engaged with the protrusion.

2. An endoscopic treatment tool comprising:
an insertion section that has flexibility to enable its insertion into a channel of an endoscope;
a treatment section that is disposed on a distal end side of the insertion section, and which performs a treatment on a living body; and
an operating section that is disposed in a proximal end portion of the insertion section, and which performs an operation of the treatment section,
wherein an operating wire is inserted into the insertion section so as to be freely moved forward and backward, and
the operating wire connects the operating section to a driving portion of the treatment section, the insertion section comprising a wire coil, and
wherein a pressing member is inserted into the proximal end of the coil so as to enlarge a winding diameter of the coil, thereby forming an enlarged-diameter portion in the coil so as to be engaged with the operating section.
